(19) Europäisches Patentamt
European Patent Office
Office européen des brevets

(11) Veröffentlichungsnummer: **0 048 696**
**A2**

# (12) EUROPÄISCHE PATENTANMELDUNG

(21) Anmeldenummer: **81810383.0**

(22) Anmeldetag: **17.09.81**

(51) Int. Cl.[3]: **C 07 F 7/22**
A 01 N 55/04, A 01 N 33/12
C 07 C 87/30, C 07 C 91/26
C 07 D 303/36

(30) Priorität: **23.09.80 CH 7123/80**
**24.07.81 CH 4845/81**

(43) Veröffentlichungstag der Anmeldung:
**31.03.82 Patentblatt 82/13**

(84) Benannte Vertragsstaaten:
**AT BE CH DE FR GB IT LI NL**

(71) Anmelder: **CIBA-GEIGY AG**
**Patentabteilung Postfach**
**CH-4002 Basel(CH)**

(72) Erfinder: **Wehner, Wolfgang, Dr.**
**Wetzbach 34**
**D-6144 Zwingenberg(DE)**

(72) Erfinder: **Farooq, Saleem, Dr.**
**Im Schaiengarten 2**
**CH-4107 Ettingen(CH)**

(72) Erfinder: **Köstler, Hans-Günter**
**Gerhart-Hauptmannstrasse 11**
**D-6148 Heppenheim/Bergstrasse(DE)**

## (54) Zinnverbindungen.

(57) Organozinn-Ammoniumsalze der Formel

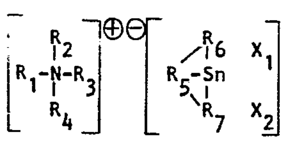 und 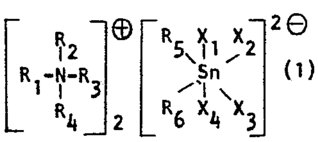 (1)

worin

$R_1$, $R_2$ und $R_3$ je Wasserstoff, Alkyl, gegebenenfalls acyliertes Halogenalkyl, Halogenhydroxyalkyl, Hydroxyalkyl oder Dihalogenalkyl oder Epoxyalkyl,

$R_4$ gegebenenfalls acyliertes Halogenalkyl, Halogenhydroxyalkyl, Hydroxyalkyl, Dihalogenalkyl, Epoxyalkyl oder Benzyl,

$R_5$ $C_1$-$C_6$-Alkyl, Cyclohexyl oder Phenyl,

$R_6$ und $R_7$ je Halogen, $C_1$-$C_6$-Alkyl, Cyclohexyl oder Phenyl und

$X_1$ bis $X_4$ je Halogen bedeuten.

Verfahren zur Herstellung dieser Verbindungen und ihre Verwendung in der Schädlingsbekämpfung werden beschrieben.

EP 0 048 696 A2

CIBA-GEIGY AG
Basel (Schweiz)

5-13072/1+2

Zinnverbindungen

Die vorliegende Erfindung betrifft Organozinn-Ammoniumsalze, Verfahren zu ihrer Herstellung und ihre Verwendung in der Schädlingsbekämpfung.

Die Organozinn-Ammoniumsalze haben die Formeln

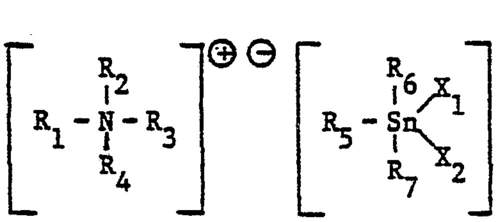 und 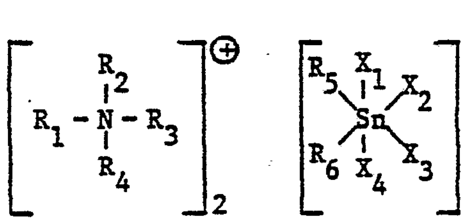 (I)

worin $R_1$, $R_2$ und $R_3$ je Wasserstoff, Alkyl, gegebenenfalls acyliertes Halogenalkyl, Halogenhydroxyälkyl, Hydroxyalkyl oder Dihalogenalkyl, Epoxyalkyl,

$R_4$ gegebenenfalls acyliertes Halogenalkyl, Halogenhydroxyalkyl, Hydroxyalkyl, Dihalogenalkyl, Epoxyalkyl oder Benzyl,

$R_5$ $C_1$-$C_6$-Alkyl, Cyclohexyl oder Phenyl,

$R_6$ und $R_7$ je Halogen, $C_1$-$C_6$-Alkyl, Cyclohexyl oder Phenyl und

$X_1$ bis $X_4$ je Halogen bedeuten.

Unter Halogen ist dabei Fluor, Chlor, Brom oder Jod, insbesondere aber Chlor oder Brom, zu verstehen.

Die Alkyl, Halogenalkyl, Halogenhydroxyalkyl, Hydroxyalkyl, Dihalogenalkyl oder Epoxyalkylgruppen bei $R_1$ bis $R_7$ können geradkettig oder verzweigt sein und haben bei $R_1$ bis $R_4$ in der Kette vorzugsweise 1 bis 20, insbesondere 1 bis 6, Kohlenstoffatome. Beispiele solcher

Gruppen sind u.a.: Methyl, Aethyl, Aethylchlorid, Aethylbromid, Propyl, Isopropyl, n-, i-, sek.-, tert.-Butyl, n-Pentyl, n-Hexyl, n-Octyl, n-Dodecyl, n-Nonadecyl-, n-Eicosyl und deren Isomere, wie z.B. 2-Aethylhexyl, 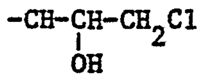 oder 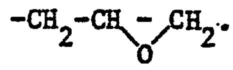.

Bevorzugt sind Verbindungen der Formel I, worin $R_1$, $R_2$ und $R_3$ je Methyl, $R_4$ Chloräthyl, Bromäthyl, 3-Chlor-2-hydroxypropyl, 2-Hydroxyäthyl oder Benzyl, $R_5$ Methyl, Cyclohexyl oder Phenyl, $R_6$ und $R_7$ je Chlor, Cyclohexyl oder Phenyl, $X_1$ Chlor und $X_2$ Chlor oder Brom bedeuten. Insbesondere bevorzugt sind aber Verbindungen der Formel I, worin $R_1$, $R_2$ und $R_3$ je Methyl, $R_4$ Chloräthyl, Bromäthyl, 3-Chlor-2-hydroxypropyl, 2-Hydroxyäthyl oder Benzyl, $R_5$, $R_6$ und $R_7$ je Cyclohexyl, $X_1$ Chlor und $X_2$ Chlor oder Brom bedeuten.

Die Organozinn-Ammoniumsalze der Formel I können nach an sich bekannten Methoden z.B. wie folgt hergestellt werden:

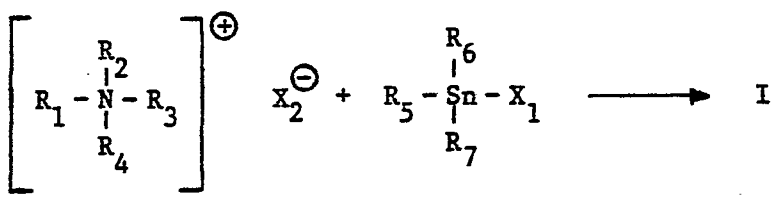

(II) (III)

In den Formeln II und III haben $R_1$ bis $R_7$, $X_1$ und $X_2$ die für die Formel I angegebene Bedeutung.

Das Verfahren wird zweckmässig bei einer Temperatur zwischen -10 bis 180°C, vorzugsweise bei 80 bis 150°C, bei normalem oder leicht erhöhtem Druck und vorzugsweise in Gegenwart eines gegenüber den Reaktionsteilnehmern inerten Lösungs- oder Verdünnungsmittels durchgeführt.

Als Lösungs- oder Verdünnungsmittel eignen sich z.B. Alkohole wie Methanol und Aethanol oder Isopropanol; Aether und ätherartige Verbindungen wie Diäthyläther, Dioxan und Tetrahydrofuran; Ketone wie Aceton, Cyclohexanon oder Methyläthylketon. Die Ausgangsstoffe der

Formeln II und III sind bekannt und können nach bekannten Methoden hergestellt werden.

Die Verbindungen der Formel I eignen sich zur Bekämpfung von verschiedenartigen Schädlingen an Tieren und Pflanzen und weisen auch eine fungizide und pflanzenregulierende Wirkung auf. Sie können somit zur Bekämpfung von Insekten, z.B. der Ordnung Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera, und von phytopathogenen Milben und Zecken der Ordnung Acarina eingesetzt werden.

Besonders hervorzuheben ist jedoch, dass die erfindungsgemässen Verbindungen der Formel I eine überraschend hohe und spezifische Wirksamkeit gegen pflanzenschädigende und tierparasitäre Milben besitzen. So können die Verbindungen der Formel I zur Bekämpfung von phytophagen Milben, z.B. der Familien Tetranychidae und Phytoptipalpidae (Spinnmilben), Tarsonemidae (Weichhautmilben) und Eriophyidae (Gallmilben), eingesetzt werden. Die Verbindungen der Formel I eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsomemus pallidus, Phyllocoptes vitis und Phyllocoptura oleivora. Mit Hilfe von Verbindungen der Formel I können auch tierparasitäre Milben, z.B. der Familien Sarcoptidae, Psoroptidae, Dermanyssidae und Demodicidae, insbesondere Räudemilben der Spezies Sarcoptes scabiei und Notoedres cati, welche sich tief in die Epidermis der befallenden Haus- und Nutztiere bis an die Nervenenden einbohren und intensive Irritationen und Schädigungen bei den Tieren verursachen, ferner die Spezies Dermanyssus gallinae und Psoroptes ovis bekämpft werden.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxyidertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyl-taurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)-äthylammoniumbromid.

Die in der Formulierungstechnik gebräuchlichen Tenside sind u.a. in folgender Publikation beschrieben:

"Mc Cutcheon's Detergents and Emulsifiers Annual" MC Publishing Corp., Ringwood, New Jersey, 1979.

Die pestiziden Zubereitungen enthalten in der Regel 0,1 bis 99 %, insbesondere 0,1 bis 95 %, Wirkstoff der Formel I, 1 bis 99,9 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele für flüssige Wirkstoffe der Formel I
(% = Gewichtsprozent)

| 1. Emulsions-Konzentrate | a) | b) | c) |
|---|---|---|---|
| Wirkstoff | 20% | 40% | 50% |
| Ca-Dodecylbenzolsulfonat | 5% | 8% | 5,8% |
| Ricinusöl-polyäthylenglykoläther (36 Mol AeO) | 5% | - | - |
| Tributylphenoyl-polyäthylenglkyoläther (30 Mol AeO) | - | 12% | 4,2% |
| Cyclohexanon | - | 15% | 20% |
| Xylolgemisch | 70% | 25% | 20% |

Aus solchen Konzentraten können durch Verdünnen mit Wasser Emulsionen jeder gewünschten Konzentration hergestellt werden.

| 2. Lösungen | a) | b) | c) | d) |
|---|---|---|---|---|
| Wirkstoff | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

Die Lösungen sind zur Anwendung in Form kleinster Tropfen geeigent.

| 3. Granulate | a) | b) |
|---|---|---|
| Wirkstoff | 5% | 10% |
| Kaolin | 94% | - |
| Hochdisperse Kieselsäure | 1% | |
| Attapulgit | - | 90% |

Der Wirkstoff wird in Methylenchlorid gelöst, auf den Träger aufgesprüht und das Lösungsmittel anschliessend im Vakuum abgedampft.

| 4. Stäubemittel | a) | b) |
|---|---|---|
| Wirkstoff | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I

(% = Gewichtsprozent)

| 5. Spritzpulver | a) | b) |
|---|---|---|
| Wirkstoff | 20% | 60% |
| Na-Ligninsulfonat | 5% | 5% |
| Na-Laurylsulfat | 3% | - |
| Na-Diisobutylnaphthalinsulfonat | - | 6% |
| Octylphenolpolyäthylenglykoläther (7-8 Mol AeO) | - | 2% |
| Hochdisperse Kieselsäure | 5% | 27% |
| Kaolin | 67% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

6. Emulsions-Konzentrat

| | |
|---|---|
| Wirkstoff | 10% |
| Octylphenolpolyäthylenglykoläther (4-5 Mol AeO) | 3% |
| Ca-Dodecylbenzolsulfonat | 3% |
| Ricinusölpolyglykoläther (36 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 7. Stäubemittel | a) | b) |
|---|---|---|
| Wirkstoff | 5% | 8% |
| Talkum | 95% | - |
| Kaolin | - | 92% |

Man erhält anwendungsfertige Stäubemittel, indem der Wirkstoff mit dem Träger vermischt und auf einer geeigneten Mühle vermahlen wird.

8. Extruder Granulat

| | |
|---|---|
| Wirkstoff | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstromgetrocknet.

9. Umhüllungs-Granulat

| | |
|---|---|
| Wirkstoff | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

10. Suspensions-Konzentrat

| | |
|---|---|
| Wirkstoff | 40% |
| Aethylenglykol | 10% |
| Nonylphenolpolyäthylenglykol-äther (15 Mol AeO) | 6% |
| Na-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 1: Herstellung des Salzes der Formel

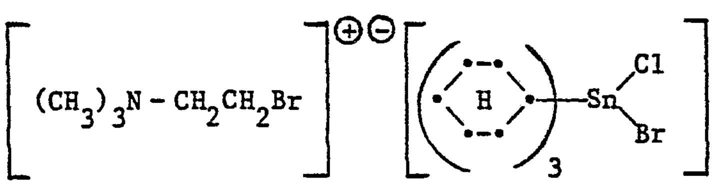

80,7 g der Verbindung der Formel

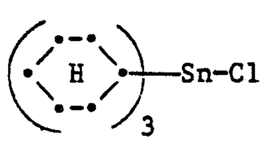

und 49,4 g des Salzes der Formel

$(CH_3)_3\overset{\oplus}{N}-CH_2CH_2Br\ \ Br^{\ominus}$ werden in einem Gemisch aus 300 ml Methanol und 550 ml Aceton gelöst. Die Lösung wird eine Stunde am Rückfluss erhitzt und eingeengt. Nach dem Ausfällen mit Aethyläther erhält man die Verbindung der Formel

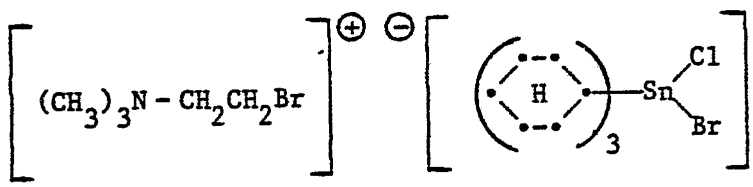

mit einem Schmelzpunkt von 205-210°C.

Auf analoge Weise werden auch folgende Verbindungen hergestellt:

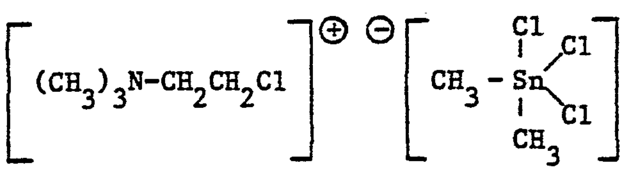 Smp.: 98-99°C

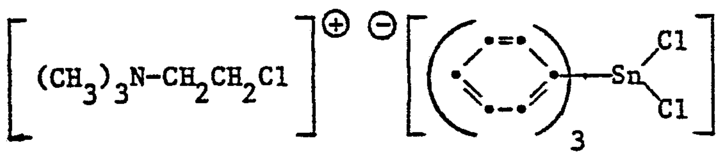 Smp.: 132-133°C

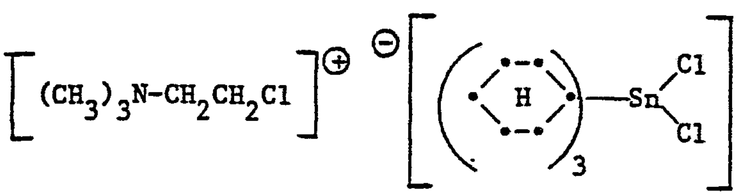 Smp.: 194-196°C

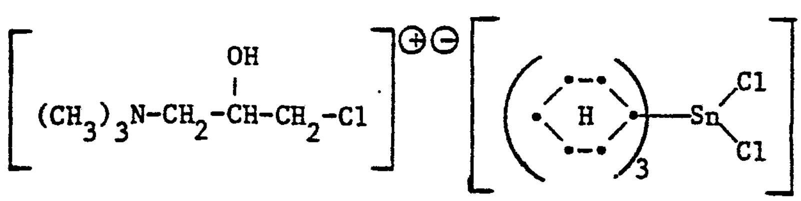 Smp.: 126-128°C

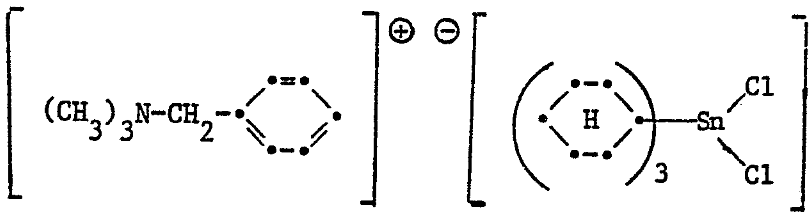

Smp.: 195-197°C

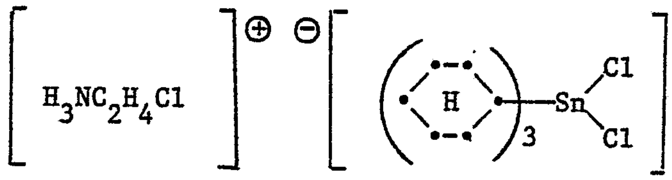

Smp.: 132-134°C

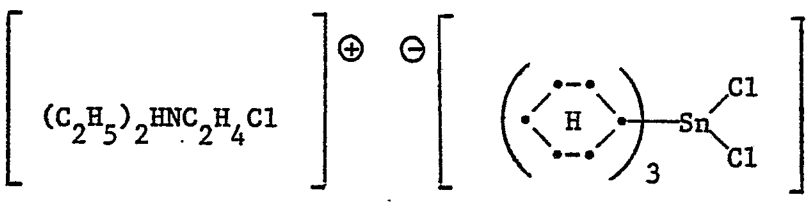

Smp.: 126-128°C

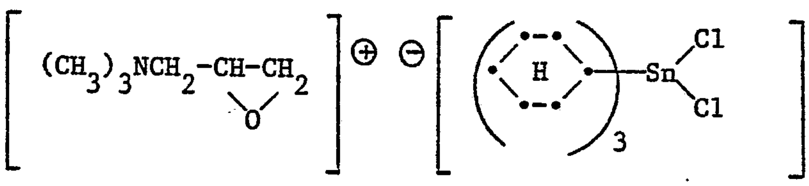

Smp.: 112-114°C

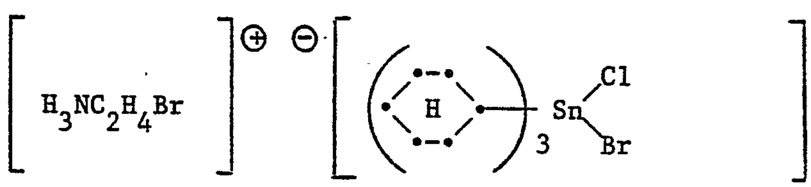

Smp.: 135-137°C

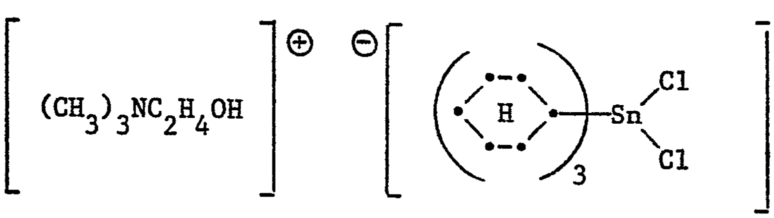

Smp.: 128-130°C

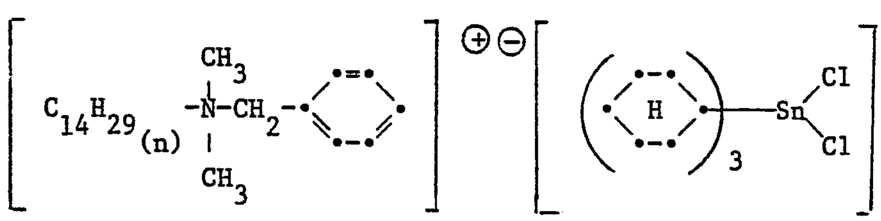

Smp.: 30-40°C (Wachs)

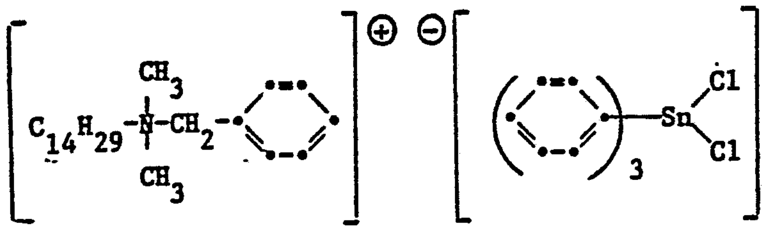

Smp.: 80-82°C

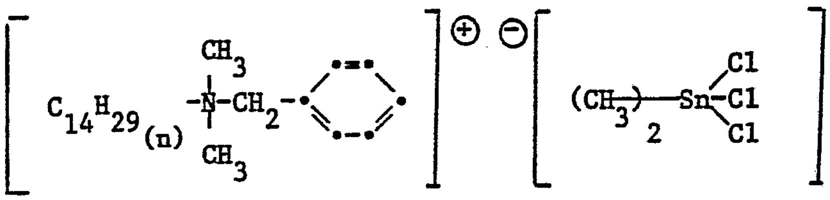

Smp.: 69-72°C

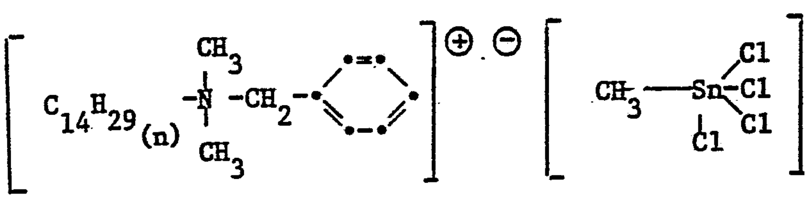

Smp.: 30-40°C (Wachs)

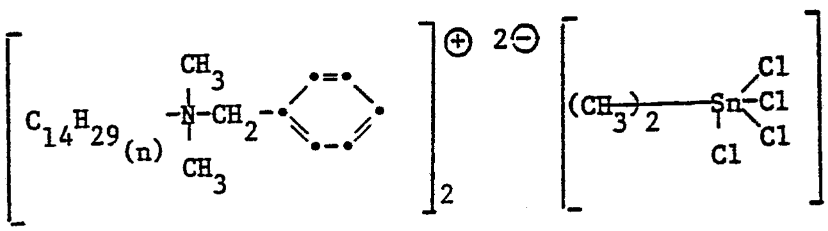

Smp.: 108-109°C

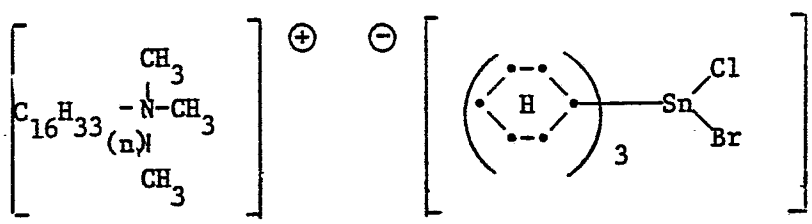

Smp.: 110 - 114°C

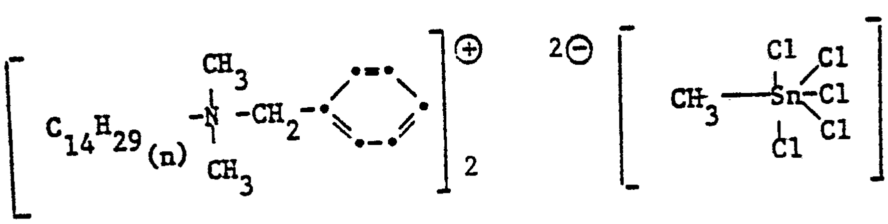

Smp.: 112-115°

Beispiel 2: Wirkung gegen pflanzenschädigende Akariden: Tetranychus urticae (OP-sensible) und Tetranychus cinnabarinus (OP-tolerant)

Die Primärblätter von Phaseolus vulgaris Pflanzen wurden 16 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt. (Die Toleranz bezieht sich auf die Verträglichkeit von Diazinon).

Die so behandelten infestierten Pflanzen wurden mit einer Versuchslösung enthaltend 25, 50, 100 oder 200 ppm der zu prüfenden Verbindung bis zur Tropfnässe besprüht.

Nach 48 Stunden und wiederum nach 7 Tagen wurden Larven unter dem Binokular auf lebende und tote Individuen ausgewertet.

Man verwendete pro Konzentration und pro Testspezies eine Pflanze. Während des Versuchsverlaufs standen die Pflanzen in Gewächshauskabinen bei 25°C.

Innerhalb der oben angegebenen Konzentrationsgrenzen zeigten Verbindungen gemäss dem Herstellungsbeispiel Wirkung gegen Larven der Spezies Tetranychus urticae und Tetranychus cinnabarninus (vgl. Tabelle).

Biologische Versuchsergebnisse

In der folgenden Tabelle sind Versuchsergebnisse auf der Basis des vorstehenden Beispiels aufgeführt, und zwar mit folgendem Bewertungsindex in Bezug auf die prozentuale Abtötung der Schädlinge:

A: 100% Abtötung bei 25 ppm Wirkstoffkonzentration
B: 100% Abtötung bei 50 ppm Wirkstoffkonzentration
C: 100% Abtötung bei 100 ppm Wirkstoffkonzentration
D: 100% Abtötung bei 200 ppm Wirkstoffkonzentration

Wirksamkeit

| Verbindungen | Tetranychus urticae Larven | Tetranychus cinnabarinus Larven |
|---|---|---|
| 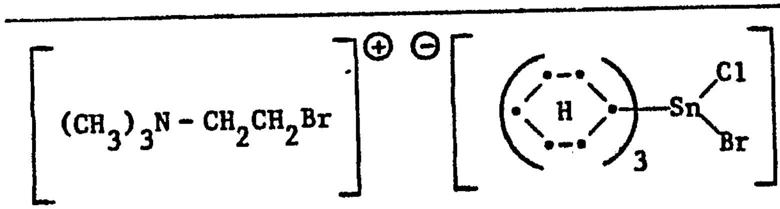 | B | A |
| 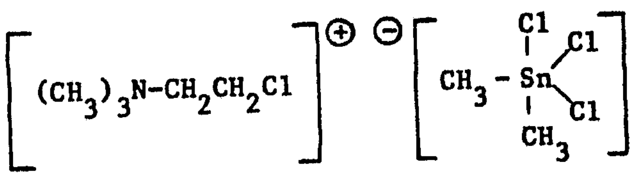 | C | B |
| 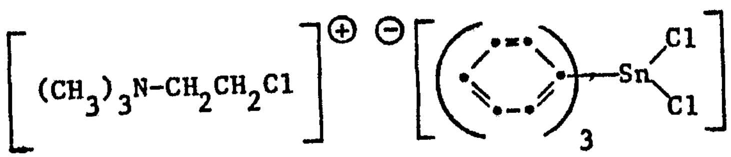 | C | B |
| 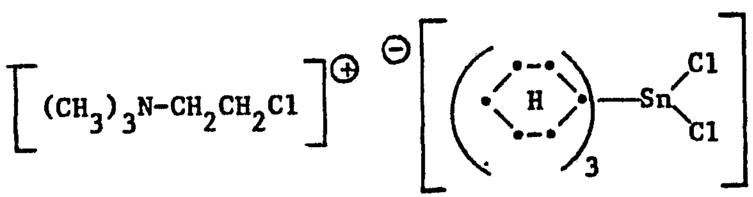 | C | B |

Wirksamkeit

| Verbindungen | Tetranychus urticae Larven | Tetranychus cinnabarinus Larven |
|---|---|---|
| 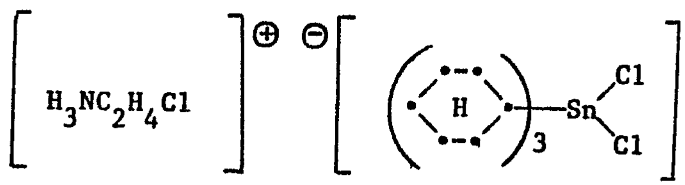 | C | - |
| 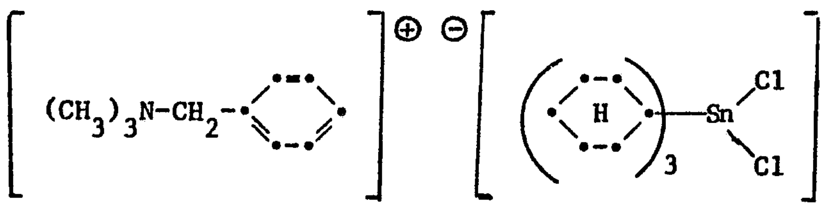 | B | A |
| 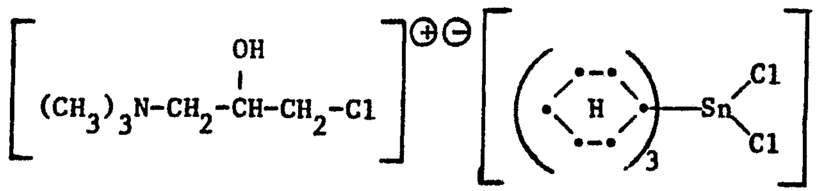 | B | A |
| 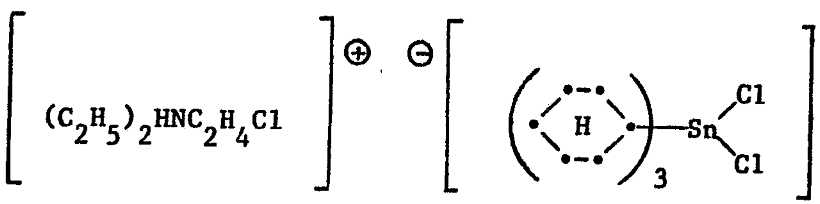 | C | - |

Wirksamkeit

| Verbindungen | Tetranychus urticae Larven | Tetranychus cinnabarinus Larven |
|---|---|---|
| 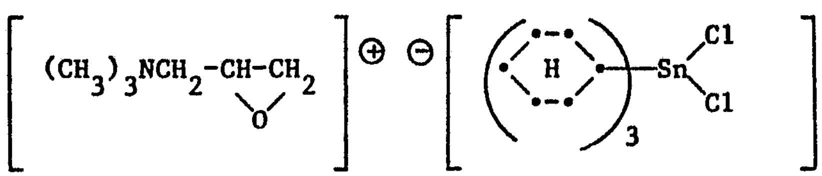 | C | - |
| 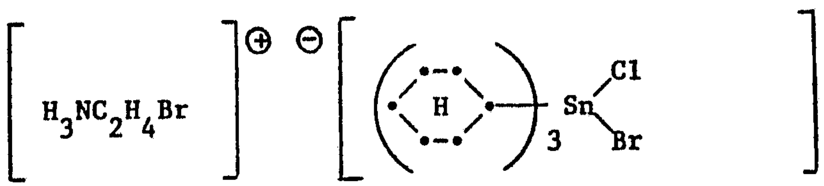 | C | - |
| 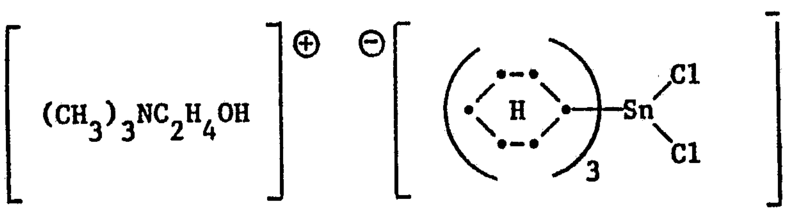 | C | - |
| 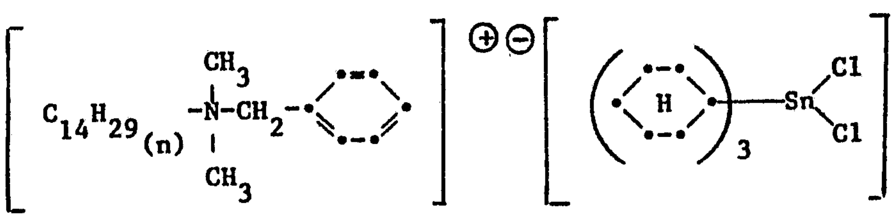 | C | B |

Wirksamkeit

| Verbindungen | Tetranychus urticae Larven | Tetranychus cinnabarinus Larven |
| --- | --- | --- |
| 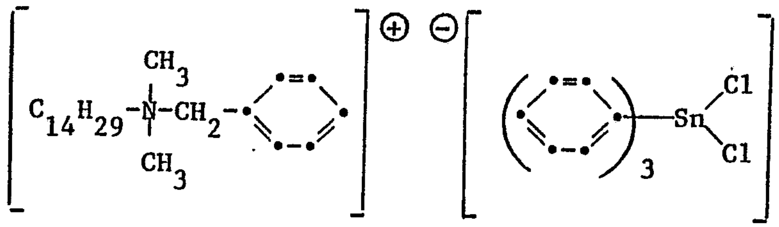 | C | B |

Patentansprüche

1. Ein Organozinn-Ammoniumsalz der Formel

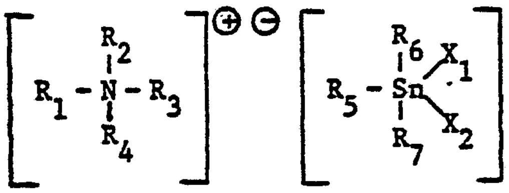 und 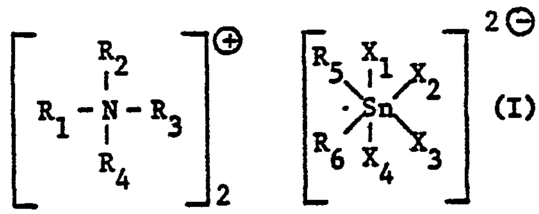 (I)

worin $R_1$, $R_2$ und $R_3$ je Wasserstoff, Alkyl, gegebenenfalls acyliertes Halogenalkyl, Halogenhydroxyalkyl, Hydroxyalkyl oder Dihalogenalkyl oder Epoxyalkyl,

$R_4$ gegebenenfalls acyliertes Halogenalkyl, Halogenhydroxyalkyl, Hydroxyalkyl, Dihalogenalkyl, Epoxyalkyl oder Benzyl,

$R_5$ $C_1$-$C_6$-Alkyl, Cyclohexyl oder Phenyl,

$R_6$ und $R_7$ je Halogen, $C_1$-$C_6$-Alkyl, Cyclohexyl oder Phenyl und

$X_1$ bis $X_4$ je Halogen bedeuten.

2. Eine Verbindung gemäss Anspruch 1, worin $R_1$, $R_2$ und $R_3$ je Methyl, $R_4$ Chloräthyl, Bromäthyl, 3-Chlor-2-hydroxypropyl, 2-Hydroxyäthyl oder Benzyl,

$R_5$ Methyl, Cyclohexyl oder Phenyl, $R_6$ und $R_7$ je Chlor, Cyclohexyl oder Phenyl,

$X_1$ Chlor und $X_2$ Chlor oder Brom bedeuten.

3. Eine Verbindung gemäss Anspruch 2, worin $R_1$, $R_2$ und $R_3$ je Methyl, $R_4$ Chloräthyl, Bromäthyl, 3-Chlor-2-hydroxypropyl, 2-Hydroxyäthyl oder Benzyl, $R_5$, $R_6$ und $R_7$ je Cyclohexyl, $X_1$ Chlor und $X_2$ Chlor oder Brom bedeuten.

4. Die Verbindung gemäss Anspruch 3 der Formel

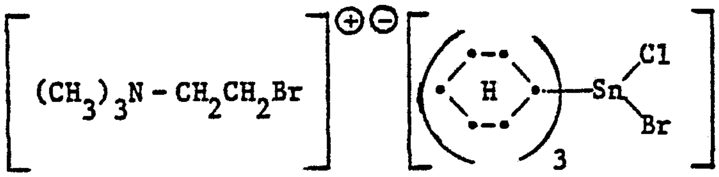

.

5. Die Verbindung gemäss Anspruch 2 der Formel

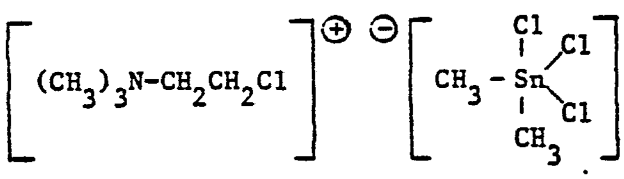

.

6. Die Verbindung gemäss Anspruch 2 der Formel

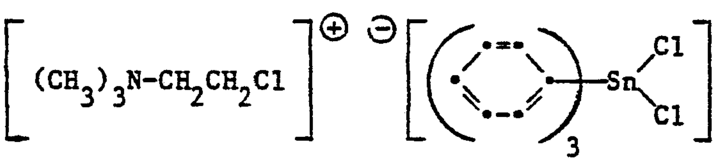

.

7. Die Verbindung gemäss Anspruch 3 der Formel

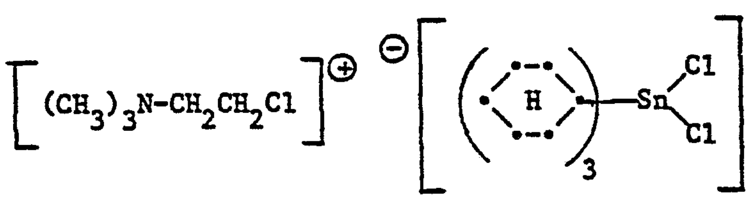

.

8. Die Verbindung gemäss Anspruch 3 der Formel

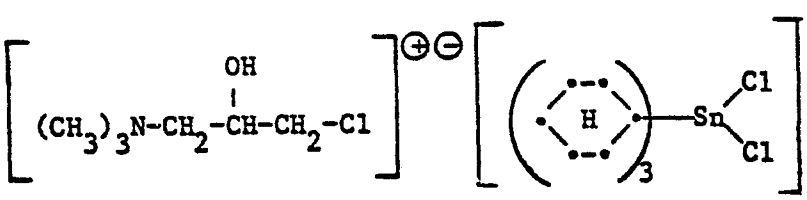

.

9. Die Verbindung gemäss Anspruch 3 der Formel

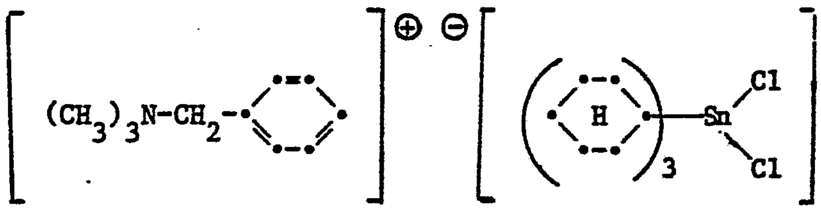

10. Die Verbindung gemäss Anspruch 1 der Formel

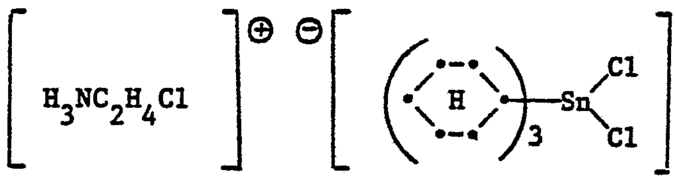

11. Die Verbindung gemäss Anspruch 1 der Formel

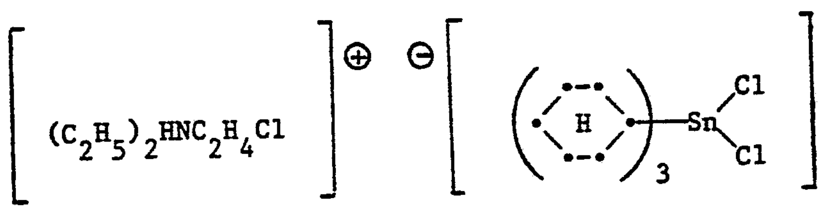

12. Die Verbindung gemäss Anspruch 3 der Formel

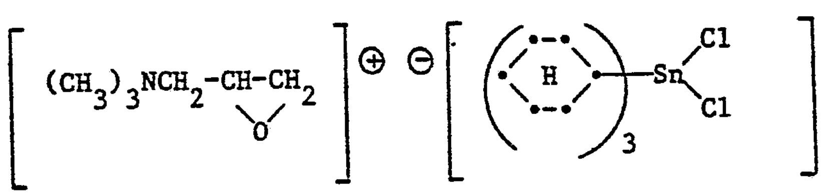

13. Die Verbindung gemäss Anspruch 1 der Formel

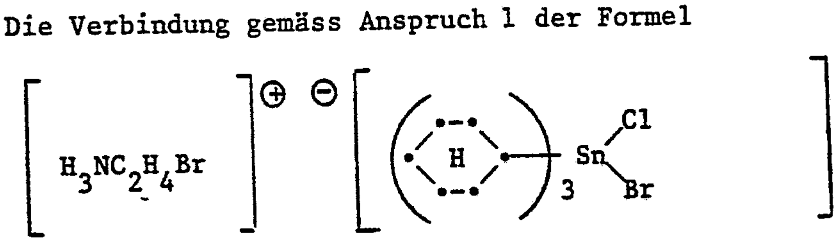

14. Die Verbindung gemäss Anspruch 3 der Formel

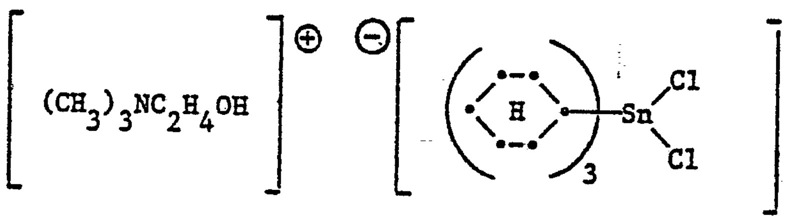

15. Die Verbindung gemäss Anspruch 1 der Formel

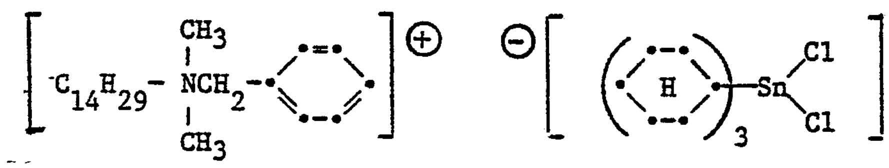

16. Die Verbindung gemäss Anspruch 1 der Formel

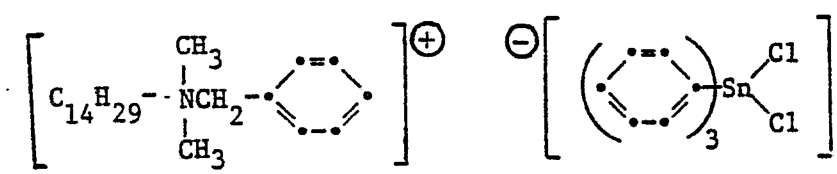

17. Ein Verfahren zur Herstellung von Verbindungen gemäss Anspruch 1, dadurch gekennzeichnet, dass man eine Verbindung der Formel

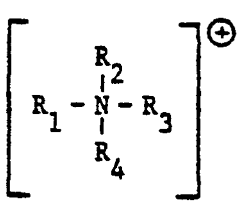 $X_2^{\ominus}$ mit einer Verbindung der Formel

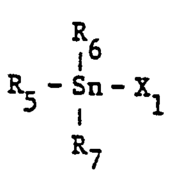 umsetzt, worin $R_1$ bis $R_6$, $X_1$ und $X_2$ die im Anspruch 1 angegebene Bedeutung haben.

18. Ein Schädlingsbekämpfungsmittel, welches als aktive Komponente eine Verbindung gemäss Anspruch 1 enthält.

19. Die Verwendung einer Verbindung gemäss Anspruch 1 zur Bekämpfung von verschiedenartigen Schädlingen an Tieren und Pflanzen sowie zur Regulierung des Pflanzenwachstums.

20. Die Verwendung gemäss Anspruch 19 zur Bekämpfung von Insekten und Vertretern der Ordnung Akarina.

21. Die Verwendung gemäss Anspruch 19 zur Bekämpfung von phytopathogenen Milben.